(19) Europäisches Patentamt
European Patent Office
Office européen des brevets

(11) Veröffentlichungsnummer: **0 176 068 B1**

# (12) EUROPÄISCHE PATENTSCHRIFT

(45) Veröffentlichungstag der Patentschrift: **06.03.91**

(51) Int. Cl.5: **C12P 13/00**, C12P 41/00, C12N 9/84, C12N 11/00, C07F 9/30, C07F 9/38

(21) Anmeldenummer: **85112018.8**

(22) Anmeldetag: **23.09.85**

## (54) Verfahren zur Herstellung der Stereoisomeren von 1-Amino-alkylphosphonsäuren oder 1-Amino-alkylphosphinsäuren.

(30) Priorität: **25.09.84 DE 3435156**

(43) Veröffentlichungstag der Anmeldung:
**02.04.86 Patentblatt 86/14**

(45) Bekanntmachung des Hinweises auf die Patenterteilung:
**06.03.91 Patentblatt 91/10**

(84) Benannte Vertragsstaaten:
**AT BE CH DE FR GB IT LI LU NL SE**

(56) Entgegenhaltungen:
**EP-A- 54 897**
**EP-A- 0 141 223**

**CHEMICAL ABSTRACTS, Band 101, Nr. 4, August 1984, Seite 488, Zusammenfassung Nr. 53353c, Columbus, Ohio, US; & HU-A-30 268 (E. MORAVCSIK) 28-03-1984; & Int. Conf. Chem. Biotechnology, Biolog. Act. Nat. Prod. (Proc.) 1. meet., Band 3(2) 1981, Seiten 221-225; J. TELEGDI et al.: "Enzymatic resolution of alpha-aminophosphonic acids" (Kat. D,A)**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Zimmermann, Gerd, Dr. rer.nat.**
**Dornheimer Ring 4**
**W-6800 Mannheim(DE)**
Erfinder: **Maier, Josef**
**Schmädlstrasse 15**
**W-8120 Weilheim(DE)**
Erfinder: **Gloger, Manfred, Dr. rer. nat.**
**Karwendelstrasse 6**
**W-8120 Weilheim(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**W-8000 München 86(DE)**

**EP 0 176 068 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung der Stereoisomeren von 1-Aminoalkylphosphonsäuren oder 1-Aminoalkylphosphinsäuren.

Peptidähnliche Derivate von 1-Aminoalkylphosphonsäuren oder auch 1-Aminoalkylphosphinsäuren besitzen antibakterielle Wirksamkeit gegenüber gram-positiven und gramnegativen Mikroorganismen und potenzieren die Aktivität von Antibiotika (z. B. von Penicillinen, Cephalosporinen und D-Cycloserin). Besondere Bedeutung in diesem Zusammenhang besitzt das Alafosfalin, ein Dipeptid aus L-Alanin und L-Aminoethylphosphonsäure.

Die von den reinen stereoisomeren Formen abgeleiteten Derivate der 1-Aminoalkylphosphonsäuren oder 1-Amino-alkylphosphinsäuren, und insbesondere die von der L-Form (R-Form, zur Nomenklatur vgl. P. Kafarski et al, Can. J. Chem. 61 (1983), 2425) abgeleiteten Derivate zeigen dabei im allgemeinen die größere biologische Aktivität (vgl. DE-PS 26 02 193, F. R. Atherton et al, Antimicrobiel Agents and Chemotherapy 15 , Mai 1979, S. 677).

Die optisch aktiven Formen der 1-Aminoalkylphosphonsäuren und -phosphinsäuren können durch chemische Racematspaltung oder durch eine asymmetrische Synthese aus optisch aktiven Vorstufen hergestellt werden (vgl. P. Kafarski et al, Can. J. Chem. 61 (1983), 2425; J. W. Huber et al, Tetrahedron Letters 33 (1979), 3049; A. Vasella und R. Veffray, Helvetica Chim. Acta 65 (1982), 1983).

Von J. Telegdi et al, Int. Conf. Chem. Biotechnol. Biol. Act. Nat. Prod. (Proc.), 1. Meet., Vol. 3 (2) (1981), S. 221-225 wird die Spaltung von racemischen N-Acetyl- oder N-Chloracetylaminophosphonsäuren in die optischen Antipoden mit Aminosäure-acylasen beschrieben. Für dieses Verfahren sind jedoch große Mengen an Enzym (50 bis 80 mg Enzym/1 mmol Substrat) und hohe Spaltzeiten (bei 37° - 40° C mehrere Stunden) erforderlich.

Aufgabe der vorliegenden Erfindung ist deshalb die Bereitstellung eines enzymatischen Verfahrens zur Herstellung der Stereoisomeren von 1-Aminoalkylphosphonsäuren oder 1-Aminoalkylphosphinsäuren, das auf einfache und wirtschaftliche Weise (breite Substratspezifität, hohe Enzymaktivität, Enzymstabilität und Stereospezifität, hohe Spaltraten, gute technische Durchführbarkeit) zu den Isomeren mit hoher optischer Reinheit führt.

Diese Aufgabe kann dadurch gelöst werden, daß man die enzymatische Spaltung der racemischen N-Acylderivate von 1-Aminoalkylphosphon- und -phosphinsäuren mit einer penicillin-G-amidase (Penicillin-G-acylase) durchführt.

Uberraschenderweise wurde nämlich gefunden, daß z. B. die N-Acetylderivate, aber auch andere N-Acylderivate von 1-Aminoalkylphosphon- und -phosphinsäuren durch Penicillin-G-amidasen mit sehr hohen Spaltraten und hoher optischer Reinheit stereoselektiv gespalten werden können; die Spaltraten lassen sich durch geeignete Auswahl der N-Acylreste beeinflussen.

Gegenstand der Erfindung ist deshalb ein Verfahren zur Herstellung der Stereoisomeren von 1-Aminoalkylphosphonsäuren oder 1-Aminoalkylphosphinsäuren durch enzymatische Spaltung ihrer racemischen N-Acylderivate und nachfolgende Deacylierung, das dadurch gekennzeichnet ist, daß man die enzymatische Spaltung mit Penicillin-G-amidase durchführt.

Aus dem Stand der Technik ist es zwar bekannt, Penicillinacylasen zur enzymatischen Spaltung einiger N-Acylderivate einzusetzen, und zwar zur Spaltung von N-Acylderivaten von alpha-Aminosäuren und N-Phenylacetylderivaten von primären Aminen und Aminoalkoholen (vgl. D. Rossi, J. Org.

Chem. 44 (1979), 2222; A. Romeo et al, Tetrahedron Letters 21 (1971), 1799), zur Abspaltung von Phenylacetyl-Schutzgruppen von in Peptid eingebautem Lysin (F. Brotnik et al, Collect. Czechoslovak. Chem. Commun. 46 (1981), 1983), zur Spaltung des Antibiotikums N-Acylthienamycin (europäisches Patent 0000931) und zur Herstellung von L-Phosphinothricin (L-2-Amino-4-methylphosphino-buttersäure) durch Spaltung des Racemats (europäische Patentanmeldung 81110474.4). Der Einsatz von Penicillin-acylasen zur enzymatischen Spaltung von N- Acylderivaten von 1-Aminoalkylphosphonsäuren oder -phosphinsäuren war bisher nicht bekannt.

Es ist aber auch bekannt, daß schon geringe Veränderungen am Aminosäureteil des Substrats zu einem starken Abfall der enzymatischen Aktivität der Penicillin-G-amidase führen (vgl. A. Plaskie et al, J. Antibiotics 31 (1978) 783); aus dem zur enzymatischen Spaltung von N-Acylderivaten von alpha-Aminosäuren und primären Aminen bekannten Einsatz von Penicillinacylase war es deshalb keinesfalls zu erwarten, daß auch N-Acylderivate von 1-Aminoalkylphosphon- und -phosphinsäuren durch enzymatische Spaltung mit Penicillinacylase mit hoher Reaktionsgeschwindigkeit (Spaltraten) und hoher optischer Reinheit stereoselektiv gespalten werden können.

Das erfindungsgemäße Verfahren weist gegenüber den N-Acyl-R,S-1-aminoalkylphosphon- und -phosphinsäuren eine sehr breite Substratspezifität auf. Die Spaltaktivität nimmt im allgemeinen mit zunehmender Kohlenstoffzahl (Kettenlänge) der 1-Aminophosphonsäure oder -phosphinsäure ab; insbesondere hängt die Spaltaktivität und die Spaltungsrate aber von der Art der Substituenten an der 1-Aminogruppe, d. h. also von der Art des Acylrestes, ab. So wird z. B. die 1-Acetamino-ethylphosphonsäure mit einer spezifischen Aktivität von 0,5 U/g umgesetzt, die 1-Phenylacetamino-ethylphosphonsäure hingegen mit einer spezifischen Aktivität von 3000 U/g. Durch geeignete Auswahl des N-Acylrestes ist es deshalb möglich, eine durch eine höhere Kohlenstoffanzahl der Aminoalkylphosphonsäuren oder -phosphinsäuren bedingte Abnahme der Aktivität auszugleichen.

Erfindungsgemäß eingesetzte Penicillin-G-amidasen (Penicillin-G-acylasen, Penicillin-amidohydrolasen) sind solche Enzyme, die Penicillin zu 6-Aminopenicillansäure spalten können. Sie werden von prokaryontischen Mikroorganismen, wie insbesondere von Escherichia coli, gebildet (M. Cole et al, Meth. Enzym. 43 - (1975) 698) und sind unter der E. C. Nr. 3.5.1.11 bekannt. Erfindungsgemäß besonders bevorzugt ist Penicillin-G-amidase aus E. coli, DSM 1900 (ATCC 11105).

Die erfindungsgemäß verwendete Penicillin-G-amidase kann als freies, wasserlösliches Enzym, z. B. als Lyophilisat, oder als immobilisiertes Enzym in wasserunlöslicher Form eingesetzt werden.

Die Substratkonzentrationen liegen im allgemeinen zwischen 0,1 mol/l und der Löslichkeitsgrenze im wäßrigen oder wäßrig-organischen Reaktionsmedium. Als wäßrig-organisches Reaktionsmedium kann ein für Enzymreaktionen übliches wäßrig-organisches Reaktionsmedium verwendet werden, insbesondere ein solches, das neben Wasser vorzugsweise ein mit Wasser mischbares oder im Wasser gut lösliches organisches Lösungsmittel, insbesondere niedere Alkohole, wie z. B. Ethanol, enthält. Vorzugsweise wird ein wäßriges Reaktionsmedium verwendet.

Die Reaktionstemperatur liegt vorzugsweise zwischen 20 und 60 °C, wobei eine Temperatur von 37 °C besonders bevorzugt ist; die Reaktionsdauer hängt insbesondere von der Enzymaktivität, von der Enzym- und Substratkonzentration und der Reaktionstemperatur ab; in der Regel wird bei einer Reaktionsdauer zwischen 2 und 120 Stunden gearbeitet. Das erfindungsgemäß eingesetzte Enzym ist bei pH-Werten von ca. 5,0 bis 8,5 ausreichend aktiv, wobei der optimale pH-Wert bei 7 liegt. Es wird deshalb vorzugsweise bei pH-Werten von 5,5 bis 8,5, und insbesondere bei pH = 7 gearbeitet. Die Reaktion kann dabei ohne oder in Gegenwart eines geeigneten Puffers, wie z. B. eines Phosphatpuffers, durchgeführt werden; die pH-Werte werden zweckmäßigerweise mit einem Autotitrator-System kontrolliert.

Insbesondere für größere Ansätze im technischen Maßstab und aus wirtschaftlichen Gründen (mehrfache Verwendung des Enzyms, Stabilität) ist es zweckmäßig, das Enzym in immobilisierter Form einzusetzen; die Reaktion kann dann in einem für immobilisierte Enzyme geeigneten Reaktor (z. B. Säulenreaktor oder Batch-Reaktor, vgl. z. B. H. D. Gräf, Pharmazie in unserer Zeit, 6 (1977), 43) kontinuierlich, z. B. im Säulenverfahren, oder diskontinuierlich (ansatzweise) durchgeführt werden. Als immobilisiertes Enzym eignet sich z. B. trägergebundene Penicillin-G-amidase (Boehringer Mannheim GmbH). Die Penicillin-G-amidase ist unter den Reaktionsbedingungen mehrere Wochen biologisch und mechanisch stabil und kann deshalb wiederholt in Reaktoren eingesetzt werden; nach 40 Tagen (0,2 mol/l Substratlösung, 37 °C) war der Aktivitätsverlust <5 %. Als Reaktoren werden Batch-Reaktoren, wie z. B. Rührkesselreaktoren, bevorzugt, weil bei diesen Reaktoren eine optimale pH-Kontrolle leicht technisch durchführbar ist.

Die enzymatische Spaltung kann analytisch leicht auf bekannte Weise verfolgt werden; die Spaltung der N-Acetylderivate wird vorzugsweise durch eine diskontinuierliche enzymatische Acetatbestimmung verfolgt, die Spaltung anderer N-Acylderivate, z. B. der Chloracetyl- oder der Phenylacetylderivate, vorzugsweise mit Hilfe der bekannten Ninhydrinmethoden zur Bestimmung freier Aminogruppen. Die stereoselektive Spaltung läßt sich über eine polarimetrische Messung der isolierten R-1-Aminophosphonsäuren und über eine 50%ige Approximation des Umsatzes nachweisen. Technische Reaktoren können über eine Feinmessung der Drehwertsänderung in der Reaktionslösung gesteuert werden.

Die Aufarbeitung der Reaktionsmischungen der enzymatischen Spaltung (Spaltansätze) kann z. B. nach den beiden folgenden Methoden erfolgen: 1. Die mit Essigsäure angesäuerte Reaktionsmischung wird eingedampft und die S-1-Acylamino-alkylphosphonsäure und die durch enzymatische Hydrolyse des Acylrestes freigesetzte Carbonsäure mit Ethanol extrahiert. Als Rückstand verbleibt die in Ethanol unlösliche R-1-Aminoalkylphosphonsäure. 2. Der Spaltansatz wird an einem stark sauren Ionenaustauscher in der $H^+$ -Form chromatographiert. Dabei werden mit Wasser als Elutionsmittel nacheinander die S-1-Acylamino-alkylphosphonsäure zusammen mit der Carbonsäure des Acylrestes und anschließend die R-1-Aminoalkylphosphonsäure in reiner Form eluiert. Die Carbonsäure des Acylrestes kann von der S-1-Acylaminoalkylphosphonsäure durch einfache Extraktion mit organischen Lösungsmitteln abgetrennt werden. Die Phosphonsäure verbleibt in der Regel in der wäßrigen Phase und kann durch Eindampfen isoliert werden. Die N-acylierte S-1-Aminoalkylphosphonsäure wird deacyliert, z. B. durch Kochen mit 6 mol/l wäßriger HCl. Die Aminophosphonsäure wird in bekannter Weise durch Behandeln der Lösung des Hydrochlorids in

Ethanol mit Propylenoxid oder durch Chromatographie an einem stark sauren Ionenaustauscher in der $H^+$ - Form isoliert.

In analoger Weise kann die Aufarbeitung eines Spaltansatzes einer 1-Acylamino-alkylphosphinsäure durchgeführt werden.

Die R- und S-Isomeren der 1-Aminoalkylphosphonsäuren und 1-Aminoalkylphosphinsäuren werden nach dem erfindungsgemäßen Verfahren mit einer hohen optischen Reinheit ( >95 %) erhalten.

Das erfindungsgemäße Verfahren eignet sich insbesondere zur Herstellung der Stereoisomeren von 1-Aminoalkylphosphonsäuren der Formel

$$R_2 - \overset{NH_2}{\overset{|}{CH}} - \overset{O}{\overset{\|}{P}}(OH)_2$$

worin $R_2$ einen verzweigten oder vorzugsweise geradkettigen Alkylrest mit 1 bis 6, insbesondere 1 bis 4 Kohlenstoffatomen darstellt, der gegebenenfalls auch substituiert sein kann, z. B. durch Halogen, Hydroxy, Alkoxy mit 1 bis 3 Kohlenstoffatomen, Phenyl und/oder Phenoxy, oder einen Phenylrest bedeutet. Der Rest $R_2$ ist z. B. Methyl, Hydroxymethyl, Ethyl, Propyl, Isopropyl, Butyl, Isobutyl, tert.-Butyl, Phenyl oder Benzyl. Für die analog aufgebauten 1-Aminoalkylphosphinsäuren kann $R_2$ die gleichen Bedeutungen besitzen.

In den als Ausgangsprodukte für die erfindungsgemäße enzymatische Spaltung verwendeten racemischen 1-Acylamino-alkylphosphonsäuren oder -phosphinsäuren ist der Acylrest $R_1$-CO- insbesondere ein solcher, in dem $R_1$ eine verzweigte und vorzugsweise geradkettige Alkylgruppe mit 1 bis 6 Kohlenstoffatomen darstellt, die gegebenenfalls durch Halogen, Hydroxy, Alkoxy mit 1 bis 3 Kohlenstoffatomen, Phenyl, Phenoxy und/oder Thienyl substituiert sein kann, wobei eine Phenyl- oder Phenoxygruppe auch noch substituiert sein kann, z. B. durch Alkyl mit 1 bis 3 Kohlenstoffatomen, Hydroxy, Nitro, Amino, Halogen und/oder Alkoxy mit 1 bis 3 Kohlenstoffatomen. Der Rest $R_1$ ist z. B. Methyl, Butyl, Chlormethyl, Benzyl, Phenoxymethyl, 2-Methylbenzyl, 4-Nitrobenzyl, 4-Hydroxybenzyl, 4-Aminobenzyl, Thienyl-(2)-, 4-Chlorbenzyl oder 4-Methoxybenzyl.

Die als Ausgangsprodukte verwendeten 1-Acylamino-alkylphosphonsäuren lassen sich nach an sich aus der Peptidchemie bekannten Methoden durch Umsetzung der entsprechenden 1-Aminoalkylphosphonsäuren mit aktivierten Carbonsäurederivaten oder Carbonsäuren in Gegenwart eines Kondensationsmittels herstellen. Die 1-Acylamino-alkylphosphinsäuren sind auf analoge Weise erhältlich. Als aktivierte Carbonsäurederivate kann man z. B. Säurechloride, symmetrische Anhydride oder gemischte Anhydride mit Kohlensäuremonoalkylestern, aktive Ester, wie z. B. p-Nitrophenylester, 2,4,5-Trichlorphenylester, N-Hydroxysuccinimid oder 1-N-Hydroxybenzotriazolester verwenden. Als Kondensationsmittel kommen hauptsächlich Carbodiimide, z. B. Dicyclohexylcarbodiimid und N,N'-Carbonyldiimidazol, in Betracht.

Die Aminogruppe der zwitterionischen 1-Aminoalkyl-phosphonsäure wird zweckmäßigerweise durch Neutralisation der Phosphonsäuregruppe mit einer Alkalimetallbase, z. B. NaOH, oder einer tertiären Aminbase, z. B. Triethylamin, freigesetzt. Man kann auch die Aminoalkylphosphonsäuren in Form ihrer Alkylester oder Trialkylsilylester mit einer aktivierten Carbonsäure acylieren. Nach der Acylierungsreaktion können die Phosphonsäurealkylester nach bekannten Methoden durch Umsetzung mit Bromwasserstoff in Eisessig oder Trimethyljod- oder Bromsilan bzw. Trimethylchlorsilan/Natriumjodid gespalten werden. Trialkylsilylester werden sehr einfach bereits durch Wasser hydrolysiert.

Die Acylierungsreaktion kann je nach der Hydrolysestabilität der Reaktionskomponenten in Wasser bzw. Wasser/ Alkohol-Mischungen oder in inerten organischen Lösungsmitteln wie z. B. in Methylenchlorid, Aceton, Acetonitril, Tetrahydrofuran, Dimethylformamid usw. durchgeführt werden. Entsprechendes gilt für die Phosphinsäurederivate.

Die racemischen 1-Aminoalkylphosphonsäuren und -phosphinsäuren sind bekannt oder können nach an sich bekannten Verfahren hergestellt werden (vgl. z. B. Synthesis 883 (1977), 479 (1978); Pol. J. Chem., 52 , 2271 (1978).

Die nachfolgenden Beispiele erläutern die Erfindung näher.

**Beispiele**

1. Synthese der 1-Acylamino-alkylphosphonsäuren

**Beispiel 1**

10 g R,S-1-Aminoethylphosphonsäure werden in 60 ml Wasser gelöst und unter Rühren bis pH 9 mit 4 mol/l NaOH versetzt. Dann werden unter Eiskühlung 12,3 g phenylessigsäurechlorid zugetropft, wobei der pH der Reaktionsmischung durch Zugabe von 4 mol/l NaOH auf 9 gehalten wird. Das Eisbad wird entfernt und das Gemisch bei Raumtemperatur gerührt, bis der pH-Wert ohne weitere Zugabe von NaOH bei pH 9 stehen bleibt. Die alkalische Phase wird mit Ether extrahiert und die Wasserphase schließlich mit Salzsäure angesäuert. Es fallen feine, weiße Kristalle aus, die abgesaugt und getrocknet werden. Zur Reinigung wird aus Isobutylmethylketon umkristallisiert.

Man erhält 9,5 g (49%) R,S-1-Phenylacetamino-ethylphosphonsäure vom Fp. 140 - 143° (Zers.).

Die in der folgenden Tabelle 1 aufgeführten 1-Acylaminoalkylphosphonsäuren wurden analog Beispiel 1 hergestellt. Ein Teil der Verbindungen wurde aus der sauren wäßrigen Phase durch Extraktion mit Essigsäureethylester oder durch Filtration über einen stark sauren Ionenaustauscher ($H^+$-Form) isoliert (vgl. Anmerkungen zur Tabelle 1).

**Tabelle 1** 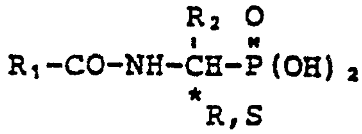

| Nr. | $R_1$ | $R_2$ | Fp. (Z = Zers.) |
|---|---|---|---|
| 1.1. | 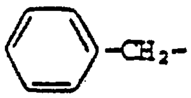 | 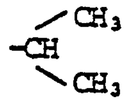 a) | 141–143° (aus Essigester-Ether) |
| 1.2. | | 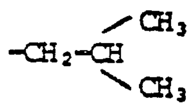 a) | 125–126° (Z) (Essigester-Ligroin) |
| 1.3. | | 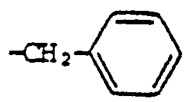 a) | 143–146° (Z) (Essigester-Ligroin) |
| 1.4. | | 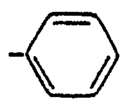 a) | 164–167° (Essigester-Ether) |
| 1.5. | | $-CH_2-OH$ b) | 208° (Z) (Aceton-Wasser) |
| 1.6. | 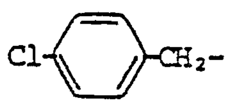 | $-CH_3$ | 163–168° (Wasser) |
| 1.7. | 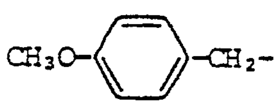 | $-CH_3$ | 180–182° (Z) |
| 1.8. | 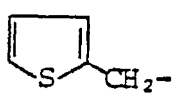 b) | $-CH_3$ | 146–150° (Wasser) |
| 1.9. | 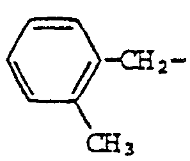 | $-CH_3$ | 153–156° (1. $H_2O$, 2. Essigester) |
| 1.10. | $CH_3-(CH_2)_4-$ b) | $-CH_3$ | 92–101° (Essigester) |

| Nr. | $R_1$ | $R_2$ | Fp. (Z = Zers.) |
|---|---|---|---|
| 1.11 | 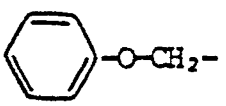 a) | $-CH_3$ | 123-125° (Z) (Essigester-Ligroin) |
| 1.12 | $Cl-CH_2-$ b) | $-CH_3$ | 101-105° (Z) |

a) Das Produkt wird mit Essigsäureethylester aus der wäßrigen, sauren Phase extrahiert.
b) Die angesäuerte wäßrige Phase wird über einen stark sauren Ionenaustauscher (DOWEX®50, $H^+$-Form) filtriert und das Produkt mit Wasser eluiert.

**Beispiel 2**

In Analogie zu Beispiel 1 erhält man unter Verwendung von R,S-1-Aminoethyl-methylphosphinsäure R,S-1-Phenylacetaminoethyl-methylphosphinsäure, die durch Extraktion der angesäuerten, wäßrigen Phase mit Essigsäureethylester isoliert und durch Umkristallisation aus Ethanol gereinigt wird. Fp. 173 - 175° C.

**Beispiel 3**

4,3 g alpha-Aminobenzylphosphonsäure werden mit 11,5 ml Eisessig und 4,7 g Acetanhydrid unter Rühren 2 Stunden auf 130° C erhitzt. Das Reaktionsgemisch wird eingedampft, in Wasser gelöst, 20 Minuten auf dem Wasserbad erhitzt und erneut eingedampft. Der verbleibende Sirup wird in Ethanol in der Hitze gelöst und die Substanz durch Zugabe von viel Ether ausgefällt. Nach dem Abkühlen wird abgesaugt. Das Rohprodukt wird aus Ethanol/Ether umkristallisiert. Man erhält 4 g (77 %) R,S-alpha-Acetamino-benzylphosphonsäure vom Fp. 205 - 207° C.

**Beispiel 4**

2,3 g R,S-1-Amino-2-methyl-propylphosphonsäure werden in 30 ml Wasser gelöst. Durch Zugabe von 4 mol/l NaOH wird der pH-Wert der Lösung auf 9 gestellt und daraufhin 4,5 g Acetanhydrid zugetropft. Der pH-Wert wird durch Zugabe von 4 mol/l NaOH auf 9 gehalten. Die Reaktionslösung wird über 300 ml stark sauren Ionenaustauscher (DOWEX®50, $H^+$-Form) filtriert (Elutionsmittel Wasser). Die produkthaltigen Fraktionen werden eingedampft und der Rückstand aus Ethanol/Ether umkristallisiert. Man erhält 1,8 g (73 %) R,S-1-Acetamino-2-methyl-propylphosphonsäure vom Fp. 178 - 180° C.

**Beispiel 5**

3,04 g p-Hydroxyphenylessigsäure und 2,02 g N-Methylmorpholin werden in einem Gemisch aus 100 ml Methylenchlorid und 10 ml Dimethylformamid gelöst. Die Lösung wird auf -15° gekühlt und tropfenweise mit 2,73 g Chlorameisensäureisobutylester versetzt. Man rührt die Reaktionsmischung 30 Minuten bei -15° und tropft dann eine Lösung des R,S-N-Trimethylsilylaminoethyl-phosphonsäure-bistrimethylsilylester zu. Das Gemisch wird je 1 Stunde bei -15°, 0° und Raumtemperatur gerührt. Dann wird zur Trockene eingeengt und der Rückstand in Wasser aufgenommen. Man stellt den pH-Wert durch Zugabe von etwas Salzsäure auf 2 ein, extrahiert mit Ether und anschließend mit n-Butanol. Die Butanolphase wird eingeengt

und der Rückstand in 1 l Ionenaustauscher DOWEX 50® $H^+$-Form mit Wasser chromatographiert. Die produkthaltigen Fraktionen werden eingedampft und zur Reinigung aus Isopropanol/Ligroin umkristallisiert. Man erhält 38 g (73 %) R,S-1-(p-Hydroxyphenylacetamido)-ethylphosphonsäurevom Fp. 185-188° (Z.).

Den verwendeten N-Trimethylsilylaminoethylphosphonsäure-bistrimethylsilylester stellt man durch 15-minütiges Erhitzen von 3 g 1-Aminoethylphosphonsäure mit 9,4 ml Trimethylchlorsilan und 10,5 ml Triethylamin in 100 ml Methylenchlorid her. Diese Lösung wird direkt für die Acylierungsreaktion verwendet.

**Beispiel 6**

3,62 g p-Nitrophenylessigsäure und 2,42 g N-Hydroxysuccinimid werden in einer Mischung aus 20 ml Tetrahydrofuran und 20 ml Methylenchlorid gelöst und bei -10° mit einer Lösung von 4,54 g Dicyclohexylcarbodiimid in 10 ml Methylenchlorid versetzt. Man rührt das Gemisch 1 Stunde bei 0° und 3 Stunden bei Raumtemperatur. Der ausgefallene Niederschlag wird abfiltriert und mit heißem Essigsäureethylester digeriert. Das Filtrat wird eingedampft, der Rückstand mit Ethanol aufgekocht und die Lösung abgekühlt.

Das auskristallisierte Produkt wird abgesaugt. Man erhält 3,75 g p-Nitrophenylessigsäure-N-hydroxysuccinimidester, den man in 50 ml Dimethylformamid löst und bei Raumtemperatur mit einer Lösung von R,S-N-Trimethylsilylaminoethyl-phosphonsäure-bistrimethylsilylester versetzt (hergestellt aus 1,88 g R,S-1-Aminoethylphosphonsäure, 5,9 ml Trimethylchlorsilan und 6,55 ml Triethylamin, vgl. oben). Das Reaktionsgemisch wird 4 Stunden bei Raumtemperatur gerührt, eingedampft und mit Natriumbicarbonatlösung und Ether versetzt. Die Etherphase wird abgetrennt und die wäßrige Phase noch zweimal mit Essigsäureethylester extrahiert. Schließlich wird die wäßrige Phase mit conc. HCl angesäuert und im Kühlschrank aufbewahrt. Die ausgefallenen Kristalle werden abgesaugt und getrocknet. Man erhält 2,1 g (36%) R,S-1-(p-Nitrophenylacetamido)-ethylphosphonsäure, die zur weiteren Reinigung aus einer Mischung aus Methanol/Ethanol und etwas Ligroin umkristallisiert werden kann. Fp. 210-215° (Z.).

**Beispiel 7**

3 g der nach Beispiel 6 erhaltenen R,S-1-(p-Nitrophenylacetamido)-ethylphosphonsäure werden in einer Mischung aus 180 ml Wasser und 60 ml Ethanol über Pd/C als Katalysator hydriert. Während der Hydrierung kristallisiert das Produkt aus. Die Reaktionsmischung wird bis zur Auflösung des weißen Reaktionsproduktes mit $NaHCO_3$ versetzt. Vom Katalysator wird abfiltriert und das Filtrat durch Zugabe von 2 mol/l HCl auf pH 5 gestellt. Das nach einiger Zeit auskristallisierte Produkt wird abfiltriert. Man erhält 1,22 g (45 %) R,S-1-(p-Aminophenylacetamido)-ethylphosphonsäure, deren Schmelzpunkt oberhalb von 300° liegt.

2. Präparative Spaltung im Rührkesselreaktor

**Beispiel 8**

Spaltung von R,S-1-Acetamino-ethanphosphonsäure

80 g lyophilisierte trägergebundene Penicillin-G-amidase (Boehringer Mannheim GmbH) werden in 1 l schwach gepufferter 0,12 mol/l Substratlösung (TRAP 0,02 mol/l, pH = 7,0) über die Dauer der Reaktion bei 37° C kräftig gerührt (Flügelrührer), anschließend über eine Nutsche abfiltriert, gewaschen und zur weiteren Verwendung lyophilisiert.

Fig. 1 zeigt das Umsatz/Zeit-Diagramm der diskontinuierlichen Spaltreaktion. Das Filtrat wird in bekannter Weise (Ionenaustauscher, Extraktion) zur Komponententrennung weiter aufgearbeitet. Bei längeren Reaktionszeiten empfiehlt sich zur Verhinderung von Mikroorganismenbefall die Zugabe von Konservierungsmitteln oder eine Sterilfiltration der Substratlösung.

**Beispiel 9**

Spaltung von R,S-1-Phenylacetamino-ethanphosphonsäure

Die Durchführung erfolgt analog Beispiel 8, jedoch mit 500 mg lyophilisierter trägergebundener Penicillin-G-Amidase/l Substratlösung.

Fig. 2 zeigt den Reaktionsverlauf (Umsatz/Zeit-Diagramm) der diskontinuierlichen Spaltreaktion.

Zur Aufarbeitung wird 1/3 der Lösung eines Spaltansatzes aus 250 g R,S-1-Phenylacetamino-ethanphosphonsäure (Spaltrate 98,2 %) mit 150 ml Eisessig angesäuert. Die Mischung wird mit Ether extrahiert und die wäßrige Phase über 3 1 stark sauren Ionenaustauscher (DOWEX®50, $H^+$-Form) gegeben. Man eluiert mit Wasser. Die erste Fraktion (2,2 l) enthält Essigsäure. In der zweiten Fraktion (ca. 2 l) befindet sich die S-1-Phenylacetaminoethylphosphonsäure. Schließlich wird mit ca. 8 l Elutionsvolumen die R-1-Aminoethylphosphonsäure eluiert. Die Prozedur wird mit den restlichen 2/3 des Spaltansatzes wiederholt. Die Ninhydrin-positiven Fraktionen werden vereinigt und eingedampft. Das Rohprodukt wird aus Ethanol/Wasser umkristallisiert. Man erhält 60,1 g (89%) R-1-Aminoethylphosphonsäure vom Fp. 292 - 293° (Zers.).
$[\alpha]_D^{20}$ = -15,5° (c = 2,1 mol/l NaOH)

Optische Reinheit:

a) aus Drehwert: 96 % R-Enantiomer
Lit.: $[\alpha]_D^{20}$ = -16,9° (c = 2,1 mol/l NaOH; Antimicr. Ag. Chemother. 15 (1979), 677).

b) gaschromatographisch: 97,5 % R-Enantiomer
2,5 % S-Enantiomer

Die Enantiomeren wurden nach Trifluoracetylierung mit Trifluoracetanhydrid und Veresterung durch Erhitzen mit Orthoameisensäureethylester an einer chiralen Phase (Chirasil-Val) aufgetrennt.

Die S-1-Phenylacetamino-ethylphosphonsäure enthaltenden Fraktionen aus der Ionenaustauschchromatographie werden vereinigt und stark eingeengt. Die ausgefallenen Kristalle werden abgesaugt. Man erhält 58,2 g (47 %) S-1-Phenylacetamono-ethanphosphonsäure vom Fp. 156 - 157° C (Gehalt an R-Enantiomeren nach gaschromatographischer Untersuchung 0,5 - 1 %). Die Mutterlauge wird mit konz. Salzsäure 15 Stunden unter Rückfluß gekocht. Die Lösung wird eingedampft und der Rückstand in Ethanol gelöst. Man tropft Propylenoxid zu, bis ein pH von 5 bis 6 erreicht wird, kühlt ab und saugt die ausgefallenen Kristalle ab. Es ergeben sich 27,4 g (40%) S-1-Aminoethylphosphonsäure vom Fp. 284-285° (Zers.). $H_2O$-Gehalt 1,3 %;
$[\alpha]_D^{20}$ = +15° (c = 2,1 mol/l NaOH)
Optische Reinheit:

a) aus Drehwert: 95 % S-Enantiomer
Lit.: $[\alpha]_D^{20}$ = +16,8° (c = 2,1 mol/l NaOH; Antimicr. Ag. Chemother. 15 (1979), 377).

b) gaschromatographisch: 96 - 97 % S-Enantiomer
3 - 4 % R-Enantiomer
(Die Trifluoracetylierung wurde in diesem Falle mit N-Methyl-bis-trifluoracetamid durchgeführt.)

In der Tabelle 2 sind die analog den Beispielen 8 und 9 erhaltenen Spaltraten für verschiedene R,S-1-Acylaminoalkylphosphonsäuren zusammengestellt.

Tabelle 2

| Substrat | immob. Pen-G-Amidase (U/g) | Spaltrate % R | | | |
|---|---|---|---|---|---|
| | | 1 h | 24 h | 48 h | 76 h |
| 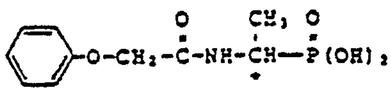 R,S | 94 | 32 | 45 | 46 | 45 |
| 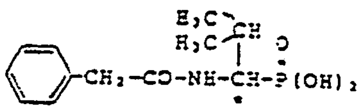 R,S | 0,4 | 2 | 12 | 18 | 32 |
| 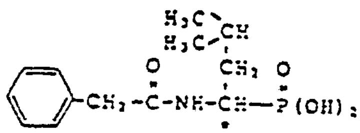 R,S | 6,3 | 6 | 19 | 28 | 45 |
| 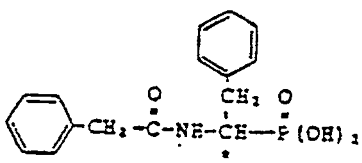 R,S | 2,8 | 4 | 15 | 25 | 38 |
| 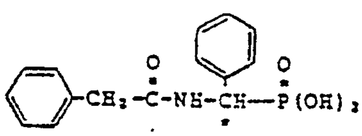 R,S | 44 | 30 | 48 | 42 | 45 |
| 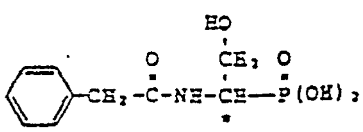 R,S | 1055 | 46 | 49 | 44 | 45 |

Tabelle 2 (Fortsetzung)

| | | | | | |
|---|---|---|---|---|---|
| 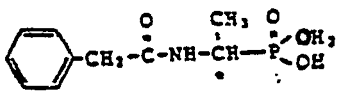 R,S | 1110 | 45 | 42 | 45 | 46 |
| 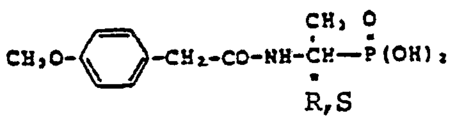 R,S | 565 | 42 | 45 | 45 | 46 |
| 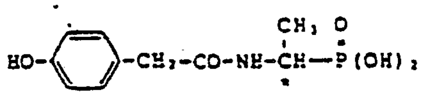 R,S | 710 | 40 | 48 | 47 | 48 |
| 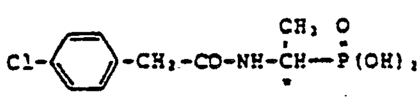 R,S | 620 | 35 | 46 | 40 | 45 |
| 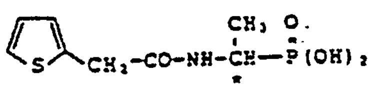 R,S | 760 | 39 | 44 | 45 | 43 |
| 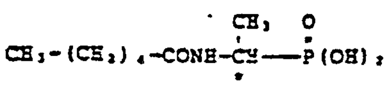 R,S | 62 | 21 | 38 | 43 | 42 |
| 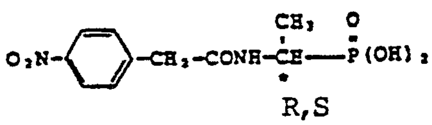 R,S | 24 | 12 | 28 | 32 | 36 |
| 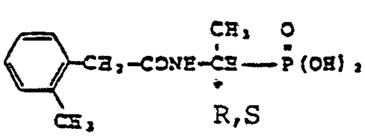 R,S | 74 | 19 | 35 | 44 | 42 |

## Ansprüche

1. Verfahren zur Herstellung der Stereoisomeren von 1-Amino-alkylphosphonsäuren oder 1-Aminoalkylphosphinsäuren durch enzymatische Spaltung ihrer racemischen N-Acylderivate und nachfolgende Deacylierung, **dadurch gekennzeichnet**, daß man die enzymatische Spaltung mit Penicillin-G-amidase durchführt.

2. Verfahren nach Anspruch 1, **dadurch gekennzeichnet**, daß man Penicillin-G-amidase aus E. coli verwendet.

3. Verfahren nach Anspruch 1 oder 2,
**dadurch gekennzeichnet**,
daß man Penicillin-G-amidase aus E. coli, DSM 1900 (ATCC 11105) verwendet.

4. Verfahren nach einem der vorhergehenden Ansprüche,
**dadurch gekennzeichnet**,
daß man die Penicillin-G-amidase in immobilisierter Form verwendet.

5. Verfahren nach Anspruch 4, **dadurch gekennzeichnet**, daß man die enzymatische Spaltung in einem Rührkesselreaktor oder Säulenreaktor durchführt.

6. Verfahren nach einem der vorhergehenden Ansprüche,
**dadurch gekennzeichnet**,
daß man die enzymatische Spaltung bei einer Temperatur von 20 bis 60° C, insbesondere bei 37° C durchführt.

7. Verfahren nach einem der vorhergehenden Ansprüche,
**dadurch gekennzeichnet**,
daß man die enzymatische Spaltung bei einem pH-Wert von 5,5 bis 8,5, insbesondere bei pH = 7, durchführt.

## Claims

1. Process for the preparation of the stereoisomers of 1-aminoalkylphosphonic acids or of 1-aminoalkylphosphinic acids by enzymatic resolution of their racemic N-acyl derivatives and subsequent deacylation, characterised in that one carries out the enzymatic resolution with penicillin G amidase.

2. Process according to claim 1, characterised in that one uses penicillin G amidase from E. coli.

3. Process according to claim 1 or 2, characterised in that one uses penicillin G amidase from E. coli, DSM 1900 (ATCC 11105).

4. Process according to one of the preceding claims, characterised in that one uses the penicillin G amidase in immobilised form.

5. Process according to claim 4, characterised in that one carries out the enzymatic resolution in a stirrer vessel or column reactor.

6. Process according to one of the preceding claims, characterised in that one carries out the enzymatic resolution at a temperature of 20 to 60° C., especially at 37° C.

7. Process according to one of the preceding claims, characterised in that one carries out the enzymatic resolution at a pH value of 5.5 to 8.5, especially at pH = 7.

## Revendications

1. Procédé de préparation des stéréo-isomères des acides 1-aminoalkylphosphoniques ou 1-aminoalkylphosphiniques par clivage enzymatique de leurs dérivés N-acylés racémiques et désacylation subséquente, caractérisé en ce qu'on effectue le clivage enzymatique au moyen de pénicilline-G-amidase.

2. Procédé selon la revendication 1, caractérisé en ce qu'on utilise de la pénicilline-G-amidase de E. coli.

3. Procédé selon la revendication 1 ou 2, caractérisé en ce qu'on utilise la pénicilline-G-amidase de E. coli, DSM 1900 (ATCC 11105).

4. Procédé selon l'une des revendications précédentes, caractérisé en ce qu'on utilise la pénicilline-G-amidase sous forme immobilisée.

5. Procédé selon la revendication 4, caractérisé en ce qu'on effectue la clivage enzymatique dans un réacteur à cuve d'agitation ou un réacteur à colonne.

6. Procédé selon l'une des revendications précédentes, caractérisé en ce qu'on effectue le clivage enzymatique à une température de 20 jusqu'à 60° C, en particulier à 37° C.

7. Procédé selon l'une des revendications précédentes, caractérisé en ce qu'on effectue le clivage enzymatique à une valeur de pH de 5,5 à 8,5, en particulier à pH = 7.

FIG.1

Spaltung von 1-Acetaminoethyl-phosphonsäure

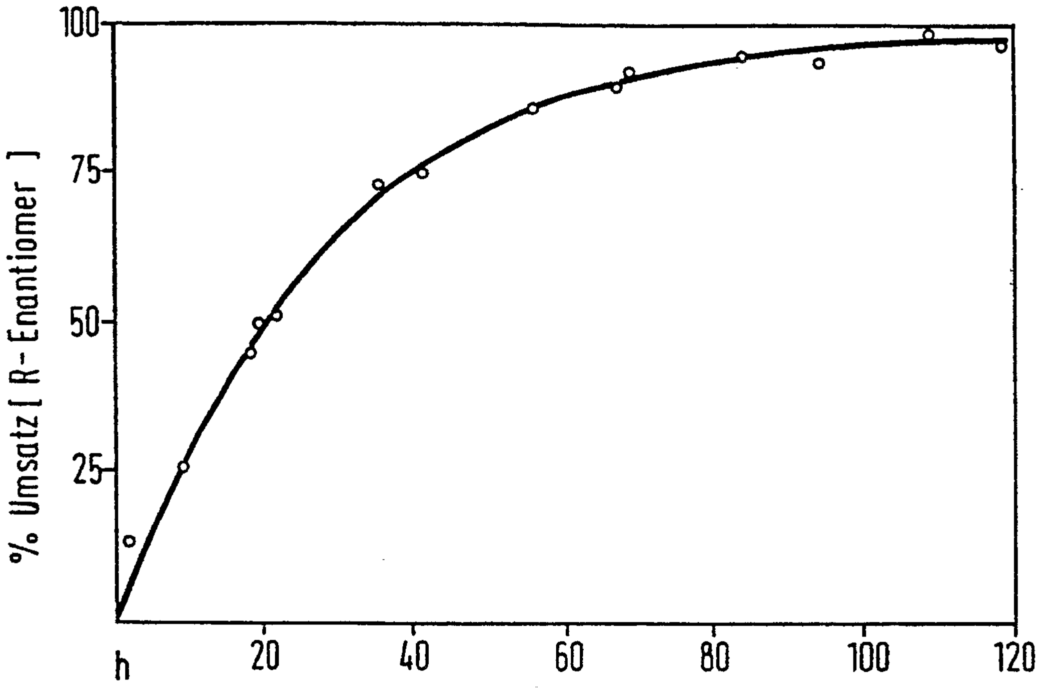


Enzym : immobil Pen.G - Amidase
Substrat - Konz. 0,12 M

FIG.2

Spaltung von 1-Phenylacetaminoethyl-phosphonsäure

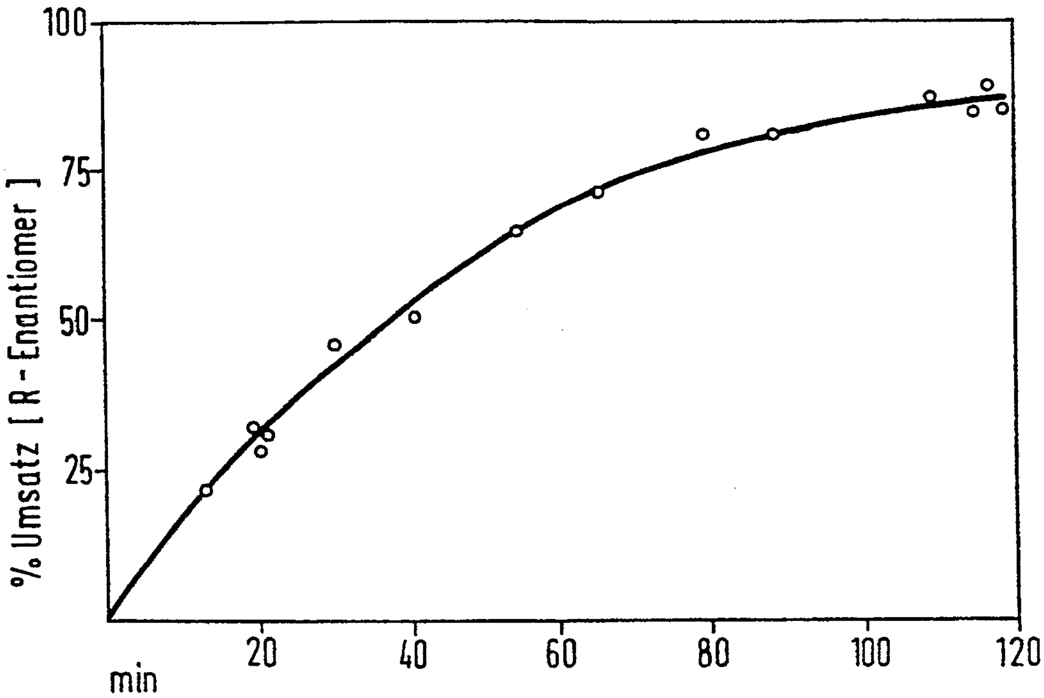


Enzym: immob. Pen.G-Amidase

Substrat-Konz. 0,1M